Europäisches Patentamt

European Patent Office

Office européen des brevets

(19)

(11) Veröffentlichungsnummer : **0 337 419 B1**

(12)

# EUROPÄISCHE PATENTSCHRIFT

(45) Veröffentlichungstag der Patentschrift :
**16.09.92 Patentblatt 92/38**

(51) Int. Cl.⁵ : **G01N 33/48,** G01N 1/22

(21) Anmeldenummer : **89106494.1**

(22) Anmeldetag : **12.04.89**

(54) **Atemanalysesystem.**

(30) Priorität : **13.04.88 DE 3812235**

(43) Veröffentlichungstag der Anmeldung :
**18.10.89 Patentblatt 89/42**

(45) Bekanntmachung des Hinweises auf die
Patenterteilung :
**16.09.92 Patentblatt 92/38**

(84) Benannte Vertragsstaaten :
**DE FR GB NL**

(56) Entgegenhaltungen :
**DE-B- 1 201 091**
**GB-A- 2 034 468**

(73) Patentinhaber : **Drägerwerk**
**Aktiengesellschaft**
**Moislinger Allee 53-55**
**W-2400 Lübeck 1 (DE)**

(72) Erfinder : **Stock, Burkhard, Dipl.Ing.Dr.**
**Sauerbruchweg 1**
**W-2400 Lübeck (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 337 419 B1

Jouve, 18, rue Saint-Denis, 75001 PARIS

## Beschreibung

Die Erfindung betrifft ein Atemanalysesystem mit einem Strömungskanal, durch den das Atemgas eines Probanden strömt und einer Analysekammer, in der das Atemgas in Verbindung zu einem Sensor steht, der einen Bestandteil des Atemgases anspricht und ein Meßsignal erzeugt.

Für die Analyse des menschlichen Atems, insbesondere zur Bestimmung der Alkoholkonzentration im Atemgas, werden neben den bekannten chemisch arbeitenden Prüfröhrchen bereits seit vielen Jahren Analysesysteme eingesetzt, die einen Sensor verwenden, der spezifisch auf den Gehalt des gesuchten Bestandteils in dem Atemgas anspricht und ein entsprechendes elektrisches Ausgangssignal erzeugt. Insbesondere werden elektrochemische Sensoren eingesetzt, wobei sich für den Zweck der Alkoholanalyse vor allem Sensoren bewährt haben, die auf einem coulometrischen Meßprinzip basieren.

Bei einem deratigen Meßverfahren wird nicht unmittelbar die Konzentration bestimmt, sondern die Menge einer an der Sensoroberfläche in einem bestimmten Zeitraum umgewandelten Species. Man spricht auch von einem "aufzehrenden Meßverfahren". Die Erfindung richtet sich insbesondere auf die Verwendung con coulometrisch oder nach einem anderen aufzehrenden Meßverfahren arbeitenden Sensoren.

Um aus der gemessenen Menge auf die Konzentration des gesuchten Stoffes im Atemgas schließen zu können, ist es erforderlich, ein definiertes Volumen des Atemgases für die Messung abzuteilen. Die entsprechenden Analysesysteme haben deswegen eine Analysekammer, die üblicherweise mit dem Strömungskanal, durch den das Atemgas des Probanden strömt, in Verbindung steht.

In der US-A-3 854 320 ist ein Atemgasanalysesystem beschrieben, bei dem die Analysekammer ein Teil des Strömungskanals ist, der permanent im Weg des Atemgases liegt. Der Sensor ist in der Analysekammer angeordnet, wird jedoch während des Ausatemvorgangs zunächst von einer entsprechenden Kappe abgedeckt. Erst gegen Ende des Ausatemvorgangs wird der Atemgasstrom unterbrochen und der Sensor durch Abheben der Kappe freigelegt, so daß die Messung durchgeführt werden kann.

Bei dem in der DE-A-29 44 444 beschriebenen Atemanalysesystem ist die Analysekammer über eine Verbindungsleitung mit dem Strömungskanal verbunden. Zum gewünschten Zeitpunkt wird eine Probe aus dem Strömungskanal in die Analysekammer gesaugt. Hierzu ist eine mit der Analysekammer verbundene Pumpeinrichtung vorgesehen, die ein in einem passenden Gehäuse kolbenartig bewegliches Element aufweist.

Um ein Atemanalysesystem der eingangs bezeichneten Art mit vereinfachtem Aufbau, zuverlässiger Funktion und einem genau definierten Volumen der Analysekammer zur Verfügung zu stellen, schlägt die Erfindung vor, daß der Strömungskanal ein Kanalelement mit einer Ausnehmung einschließt, welches Kanalelement in einem Gehäuse mit einem Einlaß und einem Auslaß für das Atemgas in mindestens zwei Positionen derartig verstellbar ist, daß in einer ersten Position die Ausnehmung mit dem Einlaß und dem Auslaß für das Atemgas in Verbindung steht, wobei die Ausnehmung einem Teil des Strömungskanals bildet und in einer zweiten Position die Ausnehmung von dem Strömungskanal getrennt und mit dem Sensor verbunden ist, wobei sie mindestens einen Teil der Analysekammer bildet.

Während sich das bewegliche Kanalelement in der ersten Position befindet, kann der Proband mit geringem Strömungswiderstand ausatmen.

Im Vergleich zu der vorbekannten Einrichtung gemäß der US-A- 3 854 320 ist es vorteilhaft, daß das Volumen des Strömungskanals, einschließlich des Abschnitts, der von der Ausnehmung des Kanalelementes gebildet wird, verhältnismäßig klein ist.

Die Ausnehmung wird durch den Atem des Probanden gespült. Für die Genauigkeit und Schnelligkeit der Messung ist es vorteilhaft, wenn das nicht gespülte Volumen der Analysekammer möglichst klein ist, d.h. die Ausnehmung sollte einen großen Teil des Gesamtvolumens der Analysekammer (mindestens 70 %, besonders bevorzugt mindestens 80 %) ausmachen. Das Verhältnis des Volumens der Ausnehmung zu dem Volumen der Analysekammer kann vorteilhafterweise auch auf ein gewünschtes Atemgas-Verdünnungsverhältnis abgestimmt werden. Dies ist zweckmäßig, wenn mit einem empfindlichen Sensor eine verhältnismäßig große Konzentration analysiert werden soll.

Gegen Ende des Ausatmungsvorgangs wird das bewegliche Kanalelement von der ersten Position in die zweite Position gebracht, wobei der Umschaltzeitpunkt mit Hilfe bekannter Maßnahmen, die nicht Gegenstand der Erfindung sind, festgelegt wird. Dadurch, daß die Ausnehmung in der zweiten Position nur noch mit der Analysekammer verbunden ist, wird das Volumen, auf das sich die Analyse bezieht, definitiv festgelegt.

Bevorzugt sind die Anordnung des Sensors in dem Gehäuse, die Anordnung des Kanalelementes in der zweiten Position und die Ausnehmung des Kanalelementes so aufeinander abgestimmt, daß sich der Sensor in der zweiten Position unmittelbar an der Ausnehmung befindet. Dadurch wird die Analysekammer praktisch durch die Ausnehmung des Kanalelementes gebildet. Dies führt zu einer Erhöhung der Meßgenauigkeit und Meßgeschwindigkeit, weil lange Diffusionswege zwischen der Ausnehmung des Kanalelementes und dem Sensor vermieden werden.

Die Erfindung wird im folgenden anhand eines in den Figuren schematisch dargestellten Ausführungsbeispiels näher erläutert; es zeigen:

Fig. 1 einen Längsschnitt durch einen Teil eines erfindungsgemäßen Atemanalysesystems in einer ersten Ausführungsform,

Figuren 2 und 3 einen Schnitt entlang der Linie II-II in Fig. 1 in zwei verschiedenen Positionen des Kanalelementes,

Fig. 4 einen Schnitt durch einen Teil einer zweiten Ausführungsform der Erfindung entlang der Schnittlinie IV-IV in Fig. 5,

Figuren 5 und 6 einen Schnitt entlang der Linie V-V in Fig. 4 in zwei verschiedenen Positionen des Kanalelementes,

Figuren 7 und 8 einen Schnitt durch einen Teil einer dritten Ausführungsform der Erfindung entlang der Linie VII-VII in Fig. 9 in zwei verschiedenen Positionen des Kanalelementes,

Fig. 9 einen Schnitt durch die Ausführungsform nach Fig. 7 entlang der Linie IX-IX.

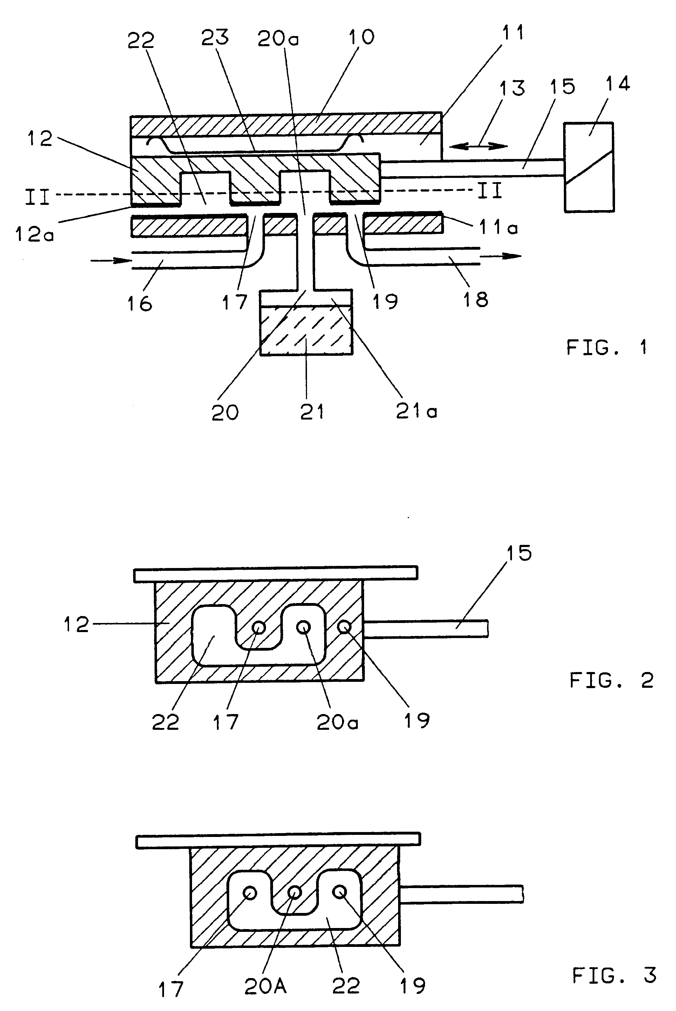

Die Figuren 1 - 3 zeigen den Teil eines Atemanalysesystems, der der Entnahme der Probe und ihrer Analyse dient.

Ein Gehäuse 10 hat eine etwa rechteckige Gehäuseausnehmung 11, in der ein als Schieber 12 ausgebildetes Kanalelement in mindestens zwei Positionen verschiebbar ist, wie durch den Pfeil 13 angedeutet wird. Die Verschiebung erfolgt mittels eines nur symbolisch dargestellten Elementantriebs 14 über eine Schubstange 15.

Das Atemgas des Probanden wird von einem nicht dargestellten Mundstück durch die Zufuhrleitung 16 zugeführt, die in einen Einlaß 17 der Gehäusewand mündet. Für die Ableitung des Atemgases ist eine Abfuhrleitung 18 vorgesehen, die von einem Auslaß 19 in der Gehäusewandung ausgeht.

Zwischen dem Einlaß 17 und dem Auslaß 19 liegt die Mündung 20a eines Verbindungskanals 20, der eine Verbindung zu einem Sensor 21 herstellt.

Der Schieber 12 hat eine Ausnehmung 22. In der in Fig. 3 dargestellten ersten Position des Schiebers fluchtet die Ausnehmung 22 mit dem Einlaß 17 und dem Auslaß 19 der Zufuhrleitung 16 bzw. der Abfuhrleitung 18 und bildet somit einen Teil des Strömungskanals 16,22,18 für das Atemgas. Gleichzeitig ist der Verbindungskanal 20 abgesperrt. Um eine zuverlässige Dichtwirkung zu erreichen, sind an den einander gegenüberliegenden Dichtflächen des Schiebers 12 und der Gehäuseausnehmung 11 Silizium-Dichtungsscheiben 12a bzw. 11a vorgesehen, und der Schieber wird in seiner Gesamtheit durch eine Feder 23 gegen die Dichtflächen gedrückt.

Zu einem geeigneten Zeitpunkt gegen Ende der Ausatemphase wird der Schieber 12 in die in den Figuren 1 und 2 dargestellte zweite Position gebracht, bei der seine Ausnehmung 22 mit der Mündung 20a des Verbindungskanals 20 fluchtet, während der Einlaß 17 und der Auslaß 19 abgesperrt sind. Nun kann die in der Ausnehmung 22 enthaltene Atemgasprobe zu dem Sensor 21 gelangen, wo ein entsprechendes Nachweissignal erzeugt wird. In dieser Stellung bilden die Ausnehmung 22, der Verbindungskanal 20 und der Gasraum 21a des Sensors 21 eine Analysekammer mit definiertem Volumen, so daß eine exakte Messung, insbesondere auch mit einem coulometrischen Sensor, möglich ist.

Bei der dargestellten Ausführungsform wird die Zufuhrleitung geschlossen, wenn das Kanalelement von der ersten in die zweite Position umgeschaltet wird. Dadurch kann der Proband nicht weiteratmen. Soweit dies nicht gewünscht ist, kann ein nicht dargestellter zweiter Strömungsweg für das Atemgas vorgesehen sein, der entweder - als permanenter Bypass - ständig geöffnet ist, oder beim Umschalten des Kanalelementes in die zweite Position geöffnet wird.

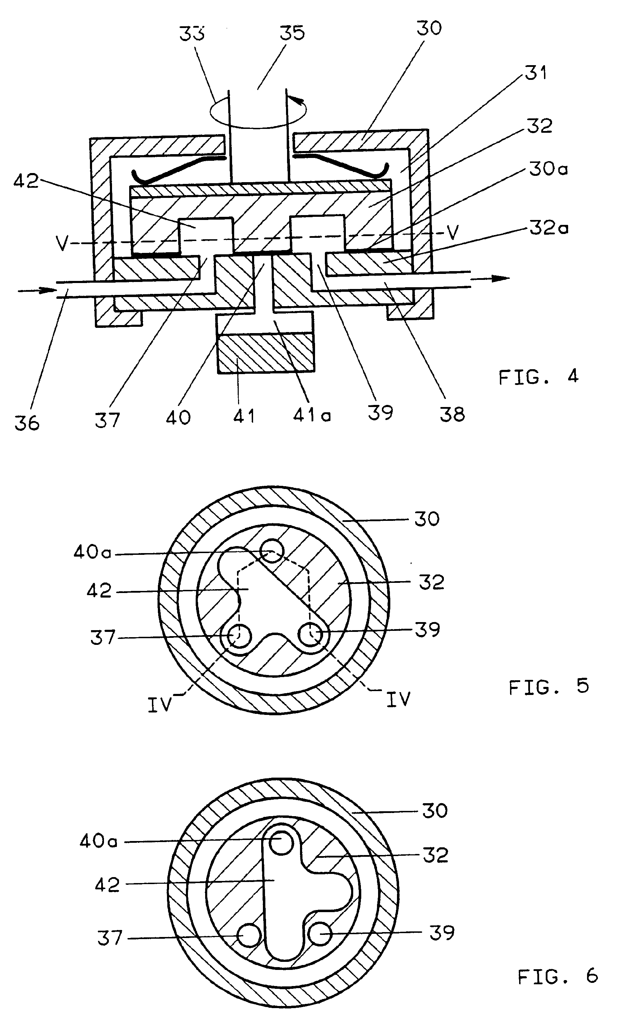

Die in den Figuren 4 - 6 dargestellte Ausführungsform ist der gemäß den Figuren 1 - 3 ähnlich, nur daß hier statt des Schiebers ein zylindrisches Kanalelement 32 verwendet wird, das in einer ebenfalls zylindrischen Ausnehmung 31 des Gehäuses 30 mit Hilfe einer Achse 35 im Sinne des Pfeils 33 drehbar ist.

Auch hier ist die Gestaltung der Ausnehmung 42 und die Positionierung des Einlasses 37 der Zufuhrleitung 36 und des Auslasses 39 der Abfuhrleitung 38 sowie der Mündung 40a des Verbindungskanals 40, der zu dem Sensor 41 führt, derartig aufeinander abgestimmt, daß in der in den Figuren 4 und 5 dargestellten ersten Position die Ausnehmung 42 mit dem Einlaß 37 und dem Auslaß 39 fluchtet, während die Mündung 40a der Verbindungsleitung 40 zu dem Sensor 41 von dem zylinderförmigen Kanalelement 32 abgesperrt wird. Die Abdichtung kann vorteilhaft dadurch sichergestellt werden, daß das Kanalelement 32 oder zumindest seine Dichtfläche 30a und/oder die entsprechende Gegenfläche 32a des Gehäuses 30 aus Graphit besteht. In der dargestellten ersten Position bildet die Ausnehmung 42 wiederum einen Teil des Strömungskanals.

In der in Fig. 6 dargestellten Stellung fluchtet die Ausnehmung 42 dagegen mit der Mündung 40a, während der Einlaß 37 und der Auslaß 39 von dem zylinderförmigen Kanalelement 32 abgesperrt werden. Hier bildet die Ausnehmung 42 deshalb wieder einen Teil der Analysekammer.

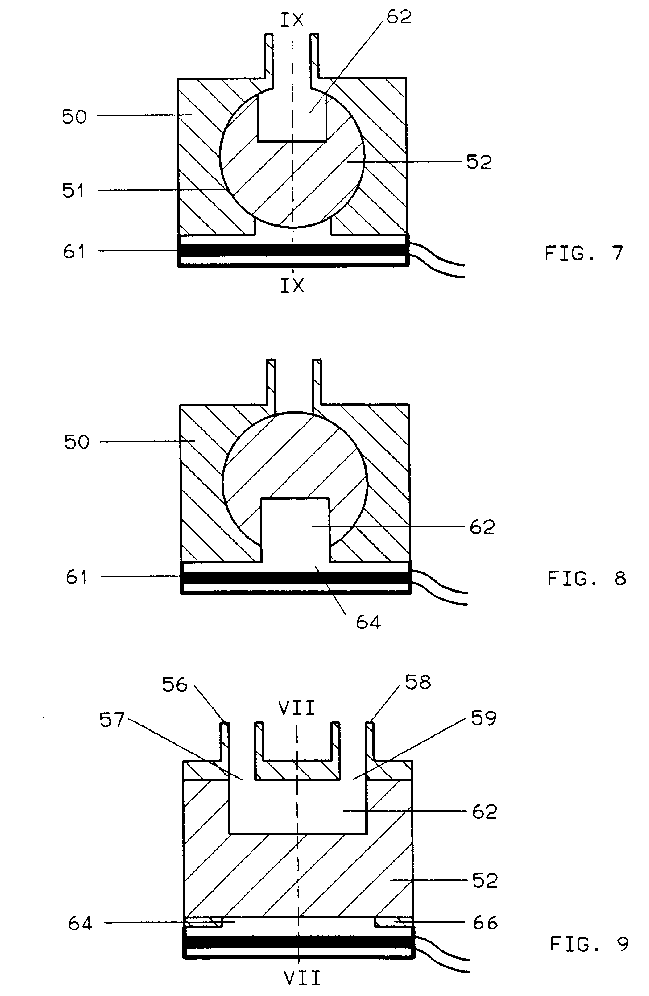

Auch bei der in den Figuren 7 - 9 dargestellten Ausführungsform wird ein um seine Zylinderachse drehbares zylinderförmiges Kanalelement 52 verwendet. Das Gehäuse 50 hat eine entsprechende zylinderförmige Gehäuseausnehmung 51. Bei diesem Ausführungsbeispiel ist die Ausnehmung 62 des Kanalelementes 52 zu dessen Zylindermantel hin geöffnet, und die Öffnung in der Zylindermantelfläche ist verhältnismäßig groß, vorzugsweise etwa ebenso groß wie die wirksame Fläche des Sensors 61.

Wiederum fluchtet in der in den Figuren 7 und 9 dargestellten ersten Position die Ausnehmung 62 des Kanalelementes 52 mit dem Einlaß 57 und dem Auslaß 59 der Zufuhrleitung 56 bzw. der Abfuhrleitung 58, von denen jeweils nur die Anschlußstutzen in der Figur dargestellt sind.

In der zweiten Position (Fig. 8) des Kanalelementes 52 liegt die Ausnehmung 62 unmittelbar gegenüber dem Sensor 61. Die Öffnung 64 in der Wand des Gehäuses 50 ist etwa ebenso groß wie die Öffnung der Ausnehmung 62 im Zylindermantel des Kanalelementes 52. Dadurch wird das Volumen der Analysekammer praktisch nur von der Ausnehmung 62 und dem geringen Volumen der Öffnung 64 gebildet. Lange Diffusionswege werden vermieden, so daß eine schnelle Messung möglich ist.

Das zylinderförmige Kanalelement 52 ist durch eine paßgenaue Führung im Gehäuse 50 derart aufgenommen, daß einerseits kein Gas während der Durchspülung zu dem Sensor 61 gelangt, andererseits das Kanalelement 52 auch noch leicht drehbar ist. Der Sensor 61 wird auswechselbar in einer Aufnahmenut 66 des Gehäuses so gehalten, daß seine wirksame Fläche sich gegenüber der Öffnung 64 befindet.

Bevorzugt ist das Gehäuse auf mindestens 34°C beheizt, um Kondensation des Wasserdampfes im Atemgas zu vermeiden.

## Patentansprüche

1. Atemanalysesystem mit einem Strömungskanal, durch den das Atemgas eines Probanden strömt und einer Analysekammer, in der das Atemgas in Verbindung zu einem Sensor steht, der auf einen Bestandteil des Atemgases anspricht und ein Meßsignal erzeugt, **dadurch gekennzeichnet, daß**
   der Strömungskanal ein bewegliches Kanalelement (12; 32; 52) mit einer Ausnehmung (22; 42; 62) einschließt, welches in einem Gehäuse(10; 30; 50) mit einem Einlaß (17; 37; 57) und einem Auslaß (19; 39; 59) für das Atemgas in mindestens zwei Positionen derartig verstellbar ist, daß
   in einer ersten Position die Ausnehmung des Kanalelementes mit dem Einlaß und dem Auslaß für das Atemgas in Verbindung steht, wobei die Ausnehmung einen Teil des Strömungskanals (16,22,18; 36,42,38; 56,62, 58) bildet und
   in einer zweiten Position die Ausnehmung des Kanalelementes von dem Strömungskanal für das Atemgas getrennt und mit dem Sensor verbunden ist, wobei sie mindestens einen Teil der Analysekammer (21a, 20,22; 41a, 40,42; 64,62) bildet.

2. Analysesystem nach Anspruch 1, **dadurch gekennzeichnet**, daß der Sensor (61) in der zweiten Position unmittelbar an der Ausnehmung (62) angeordnet ist.

3. Analysesystem nach Anspruch 1, **dadurch gekennzeichnet**, daß das Volumen der Ausnehmung (62) einen großen Teil des Gesamtvolumens der Analysekammer (62,64) ausmacht.

4. Analysesystem nach Anspruch 1, **dadurch gekennzeichnet**, daß das Volumenverhältnis der Ausnehmung (22;42;62) zu der gesamten Analysekammer (21a, 20,22;41a,40,42;62,64) einem gewünschten Atemgas-Verdünnungsverhältnis entspricht.

5. Analysesystem nach Anspruch 1, **dadurch gekennzeichnet**, daß das Kanalelement als Schieber (12) ausgebildet ist, der in einer passenden Ausnehmung des Gehäuses (10) abgedichtet verschiebbar ist.

6. Analysesystem nach Anspruch 1, **dadurch gekennzeichnet**, daß das Kanalelement (32; 52) zylinderförmig ist und das Gehäuse (30; 50) eine entsprechende Ausnehmung (31; 51) aufweist, in der das zylinderförmige Kanalelement um seine Längsachse drehbar ist.

7. Analysesystem nach Anspruch 6, **dadurch gekennzeichnet**, daß die Ausnehmung (62) des zylinderförmigen Kanalelementes (52) zum Mantel des Zylinders hin geöffnet ist und der Sensor (61) in der Wandung der Gehäuseausnehmung so angeordnet ist, daß er in der zweiten Position in der Öffnung der Ausnehmung (62) des Kanalelementes (52) liegt.

**Claims**

1. Respiratory analysis system having a flow channel through which the respiratory gas of a test person flows and an analysis chamber in which the respiratory gas communicates with a sensor which responds to a constituent of the respiratory gas and generates a measurement signal, characterised in that the flow channel includes a movable channel element (12; 32; 52) with a recess (22; 42; 62), which element in a housing (10; 30; 50) with an inlet (17; 37; 57) and an outlet (19; 39; 59) for the respiratory gas can be adjusted into at least two positions in such a way that in a first position the recess of the channel element communicates with the inlet and the outlet for the respiratory gas, with the recess forming a portion of the flow channel (16, 22, 18; 36, 42, 38; 56, 62, 58), and in a second position the recess of the channel element is separated from the flow channel for the respiratory gas and is connected with the sensor, in which case it forms at least one portion of the analysis chamber (21a, 20, 22; 41a, 40, 42; 64, 62).

2. Analysis system according to claim 1, characterised in that the sensor (61) in the second position is arranged directly at the recess (62).

3. Analysis system according to claim 1, characterised in that the volume of the recess (62) constitutes a large part of the overall volume of the analysis chamber (62, 64).

4. Analysis system according to claim 1, characterised in that the ratio of volumes of the recess (22; 42; 62) to the whole analysis chamber (21a, 20, 22; 41a, 40, 42; 62, 64) corresponds to a desired respiratory gas dilution ratio.

5. Analysis system according to claim 1, characterised in that the channel element is formed as a slide (12) which can be displaced in a sealed manner in a suitable recess of the housing (10).

6. Analysis system according to claim 1, characterised in that the channel element (32; 52) is cylindrical and the housing (30; 50) has a corresponding recess (31; 51) in which the cylindrical channel element can be rotated about its longitudinal axis.

7. Analysis system according to claim 6, characterised in that the recess (62) of the cylindrical channel element (52) is open towards the casing of the cylinder and the sensor (61) is arranged in the wall of the housing recess in such a way that in the second position it lies in the opening of the recess (62) of the channel element (52).


**Revendications**

1. Système d'analyse respiratoire comportant un canal d'écoulement à travers lequel passe le gaz respiratoire d'une personne testée ainsi qu'une chambre d'analyse dans laquelle le gaz respiratoire est en liaison avec un capteur qui réagit à un composant du gaz respiratoire et qui produit un signal de mesure, caractérisé en ce que le canal d'écoulement renferme un élément de canal (12 ; 32 ; 52) avec un évidement (22 ; 42 ; 62), lequel élément est déplaçable dans un boîtier (10 ; 30 ; 50) présentant une entrée (17 ; 37 ; 57) et une sortie (19 ; 39 ; 59) pour le gaz respiratoire, dans au moins deux positions, de manière que dans une première position, l'évidement de l'élément de canal communique avec l'entrée et la sortie du gaz respiratoire, l'évidement formant une partie du canal d'écoulement (16, 22, 18 ; 36, 42, 38 ; 56, 62, 58) et dans une deuxième position, l'évidement de l'élément de canal est séparé du canal d'écoulement du gaz respiratoire et est en liaison avec le capteur, cet évidement formant au moins une partie de la chambre d'analyse (21a, 20, 22 ; 41a, 40, 42 ; 64, 62).

2. Système d'analyse selon la revendication 1, caractérisé en ce que le capteur (61) est placé directement sur l'évidement (62), dans la deuxième position.

3. Système d'analyse selon la revendication 1, caractérisé en ce que le volume de l'évidement (62) représente une grande partie du volume total de la chambre d'analyse (62, 64).

4. Système d'analyse selon la revendication 1, caractérisé en ce que le rapport entre le volume de l'évidement (22 ; 42 ; 62) et celui de l'ensemble de la chambre d'analyse (21a, 20, 22 ; 41a, 40, 42 ; 62, 64) correspond à un rapport voulu de dilution du gaz respiratoire.

5. Système d'analyse selon la revendication 1, caractérisé en ce que l'élément de canal est un organe coulissant (12) qui peut coulisser, de façon étanche, dans un évidement adapté du boîtier (10).

6. Système d'analyse selon la revendication 1, caractérisé en ce que l'élément de canal (32 ; 52) est cylindrique et en ce que le boîtier (30 ; 50) présente un évidement (31 ; 51) correspondant dans lequel l'élément de canal cylindrique peut tourner autour de son axe longitudinal.

7. Système d'analyse selon la revendication 6, caractérisé en ce que l'évidement (62) de l'élément de canal (52) cylindrique est ouvert en direction de l'enveloppe du cylindre et en ce que le capteur (61) est placé dans la paroi de l'évidement de boîtier de manière que dans la deuxième position, il se trouve dans l'ouverture de l'évidement (62) de l'élément de canal (52).

FIG. 1

FIG. 2

FIG. 3

FIG. 4

FIG. 5

FIG. 6

FIG. 7

FIG. 8

FIG. 9